# NOUVEAU FASCICULE DE BREVET EUROPEEN

(11) **EP 0 771 324 B2**
(45) Date de publication et mention de la décision concernant l'opposition: **09.02.2011**
(45) Mention de la délivrance du brevet: 22.09.1999
(21) Numéro de dépôt: 95927599.1
(22) Date de dépôt: 14.07.1995
(51) Int. Cl.: C07K 14/75, A61L 2/18, A61K 38/36

(54) **CONCENTRE DE FIBRINOGENE ISSU DE PLASMA SANGUIN, PROCEDE ET INSTALLATION POUR SA PREPARATION**
FIBRINOGENKONZENTRATE AUS BLUTPLASMA, VERFAHREN UND ANLAGE FÜR IHRE HERSTELLUNG
CONCENTRATE OF FIBRINOGENE OBTAINED FROM BLOOD PLASMA, PROCESS AND PLANT FOR ITS PREPARATION

(30) Priorité: 14.07.1994 EP 94870121
(43) Date de publication de la demande: 07.05.1997
(73) Titulaire: CAF-DCF Département Central de Fractionnement de la Croix Rouge S.C.R.L., 1120 Brussels (BE)
(72) Inventeur: LAUB, Ruth, 1190 Bruxelles (BE); DE WAEL, Luc, 2520 Ranst (BE)
(74) Mandataire: De Clercq, Ann G. Y.
(86) Numéro de dépôt international: PCT/BE1995/000069
(87) Numéro de publication internationale: WO 1996/002571

(56) Documents cités:
- EP-A- 0 018 561
- EP-A- 0 099 445
- EP-A- 0 131 740
- EP-A- 0 311 950
- EP-A- 0 378 208
- EP-A- 0 555 135
- WO-A-86/05190
- WO-A-89/12065
- WO-A-93/05067
- DE-A- 3 001 435
- US-A- 4 295 855
- US-A- 5 099 003
- US-A- 5 252 709

## Description

### Objet de l'invention.

La présente invention concerne un procédé de préparation d'un concentré de fibrinogène issu du plasma sanguin humain ou animal.

La présente invention concerne également la composition pharmaceutique et/ou cosmétique comprenant ledit concentré de fibrinogène.

### Arrière-plan technologique à la base de l'invention.

Le fibrinogène est une protéine plasmatique d'une importance capitale dans le processus de coagulation, et ses champs d'application pharmaceutique ou cosmétique recouvrent différents domaines (cicatrisation de plaies, agent de coagulation, constituant de colles biologiques, fibrinogénémie, inhibition de conséquences opératoires et post-opératoires,

La mise à disposition de protéines du sang, telles que le fibrinogène, nécessite pour leur utilisation à des fins thérapeutiques ou non thérapeutiques des techniques de purification permettant d'obtenir des produits de haute pureté et totalement dépourvus de contaminants viraux (HIV, hépatites, parvovirus, ...) ou de molécules biologiques telles que des anticorps ou des protéases (Council Directive 65/65 EEC, 75/319/EEC & 89/381 EEC).

Aussi, différents procédés de traitement (filtration, précipitation, chromatographie d'affinité, ...) ont été proposés pour éliminer ou inactiver les contaminants du plasma et/ou de composés dérivés du plasma (facteur VIII, facteur de von Willebrand, fibronectine, ...).

Les traitements d'inactivation virale peuvent consister en un traitement thermique ou chimique.

Un traitement chimique peut par exemple consister en une inactivation virale par solvant-détergent telle que décrite dans la demande de brevet numéro EP-0 131 740.

Cependant, ces traitements thermiques ou chimiques d'inactivation virale ne permettent pas d'éliminer complètement certains contaminants viraux, en particulier certains virus non enveloppés tels que le parvovirus B19.

### Etat de la technique.

La demande de brevet PCT/FR89/00050 (WO89/12065) décrit une telle inactivation virale par solvant-détergent dans un procédé de séparation de protéines du plasma à partir d'une fraction solubilisée d'un cryoprécipité de plasma.

Selon ce procédé, on soumet la fraction d'un cryoprécipité de plasma resolubilisée dans l'eau à un traitement d'inactivation virale par solvant-détergent, puis à une séparation unique de chromatographie sur une résine échangeuse d'anions dont la matrice est un gel capable, de par ses propriétés de porosité et d'hydrophobicité, de retenir le complexe facteur VIII - facteur de von Willebrand. On récupère ensuite sélectivement chacune des protéines par des augmentations successives de la force ionique du tampon d'élution.

Le premier filtrât de la chromatographie contient principalement du fibrinogène, mais aussi de l'albumine, des immunoglobulines et les agents d'inactivation virale (Tween et TNBP).

A partir de cette solution, le fibrinogène est ensuite purifié (élimination des agents d'inactivation virale) par une nouvelle étape de chromatographie sur colonne de résine d'héparine-sépharose.

La fraction de fibrinogène recueillie est ensuite concentrée et dialysée par un système de cassette. Le produit concentré est réparti en flacons et lyophilisé.

Cependant cette technique de purification du fibrinogène présente l'inconvénient qu'elle nécessite une étape de purification complexe et coûteuse par chromatographie.

En outre, cette technique de purification ne permet pas de traiter industriellement de manière rapide des volumes importants de fractions enrichies en fibrinogène de haute pureté. De même, le concentré de fibrinogène purifié par une étape supplémentaire de chromatographie sera dépourvu de facteur XIII, ce qui réduirait son application en tant que colle biologique.

Les brevets et demandes de brevet DE-30 01 435, EP-0 311 950 et PCT/GB86/00121 (W086/05190) décrivent des procédés de purification de composés sanguins issus de fractions de plasma sanguin par précipitation à pH acide et en présence d'un acide aminé.

De tels procédés peuvent être combinés à un procédé chimique d'inactivation virale.

Néanmoins, les produits obtenus par les différentes techniques citées ne sont pas suffisamment purs.

En effet, des contaminants tels que des protéases ne sont pas éliminés par ces techniques.

De plus, le concentré de fibrinogène non délipidé obtenu ne peut être rendu soluble que très lentement.

### Buts de l'invention.

La présente invention vise à obtenir un procédé d'obtention de ce concentré de fibrinogène issu de plasma sanguin, qui ne présente pas les inconvénients de l'état de la technique cité, en particulier un procédé qui soit simple, rapide et peu coûteux, et qui permette d'obtenir de manière industrielle des volumes importants de fibrinogène hautement purifié.

La Demanderesse a réussi un procédé d'obtention du concentré de fibrinogène caractérisé en ce qu'il est dépourvu de contaminants viraux, en ce que sa pureté est supérieure à 98%, et en ce qu'il est dépourvu de protéases, caractérisé en ce que l'on soumet une fraction solubilisée de plasma comprenant du fibrinogène à un traitement chimique d'inactivation virale par addition de solvant/détergent, à deux ou plusieurs étapes de précipitation dans une solution comprenant un acide aminé, de préférence la Glycine, et à pH acide, et une ou plusieurs étape(s) de filtration du fibrinogène purifié, de préférence sur filtre de carbone activé.

En outre, la Demanderesse a réussi un procédé de l'invention, caractérisé en ce que le pH de la solution est compris entre 4.0 et 7.0, de préférence entre 5.5 et 6.5, et en ce que la concentration en acide aminé dans la solution est comprise entre 0.1 et 3.3 molaire, de préférence entre 0.5 et 1.5 molaire.

En outre, la Demanderesse a réussi un procédé de l'invention, caractérisé en ce qu'il comporte une étape thermique d'inactivation virale, de préférence par chauffage de la fraction solubilisée de plasma à une température supérieure ou égale à 80°C pendant une durée supérieure ou égale à 10 heures, et/ou une étape physique d'inactivation virale par traitement de la fraction solubilisée de plasma aux rayons ultraviolets C, dont la majorité de l'émission du rayonnement s'effectue de préférence entre 250 et 270 nm, et dans laquelle les doses d'irradiation sont de préférence de l'ordre de 250 Joules/m².

En outre, la Demanderesse a réussi un procédé de l'invention, caractérisé en ce que la fraction solubilisée du plasma est choisie parmi le groupe constitué par la fraction solubilisée d'un cryoprécipité de plasma, la fraction FI de Cohn et/ou un mélange d'entre elles.

En outre, la Demanderesse a réussi un procédé de l'invention, caractérisé en ce que la fraction solubilisée du cryoprécipité de plasma est préalablement soumise à une étape unique de chromatographie sur une résine échangeuse d'ions, comportant de préférence une matrice constituée d'un gel capable de, par ses propriétés de porosité et d'hydrophobicité de retenir le complexe facteur VIII - facteur de von Willebrand.

Egalement, la Demanderesse a réussi un procédé de l'invention, caractérisé en ce que l'on soumet préalablement la fraction solubilisée du cryoprécipité de plasma à un traitement de prépurification comprenant un traitement à l'hydroxyde d'alumine et/ou une précipitation à une température comprise entre 10 et 20°C.

De plus, la Demanderesse a réussi un composition pharmaceutique, cosmétique ou colle biologique comprenant un concentré de fibrinogène caractérisé en ce qu'il est dépourvu de contaminants viraux, en ce que sa pureté est supérieure à 98%, et en ce qu'il est dépourvu de protéases.

### Eléments caractéristiques de l'invention.

Avantageux, le procédé de l'invention a reussi à obtenir un concentré de fibrinogène qui est caractérisé par une pureté particulièrement élevée, supérieure à 95%, voire supérieure à 98%. La fraction restante dans ce concentré de fibrinogène est constituée de facteur XIII (la proportion du facteur XIII dans la fraction restante pouvant être supérieure à 30%) et d'immunoglobulines.

En outre, il peut également subsister dans ce concentré de fibrinogène une très faible concentration d'additifs chimiques (solvants / détergents) utilisés dans le procédé d'inactivation chimique de ce concentré de fibrinogène.

Cependant, les solvants / détergents détectés dans le produit final ne sont présents qu'à l'état de traces, non toxiques.

Avantageux, le procédé de l'invention a reussi à obtenir un concentré de fibrinogène qui est délipidé, c'est-à-dire qu'il peut être rapidement et aisément solubilisé en quelques minutes, de préférence en moins de 15 minutes, voire moins de 10 minutes.

Avantageux, le procédé de l'invention a reussi à obtenir un concentré de fibrinogène qui ne subit pas de coagulation après conservation pendant plus de 12 mois à 4° C.

Avantageux, le procédé de l'invention a reussi à obtenir un concentré de fibrinogène qui comporte plus de 0,001%, de préférence plus de 0,1%, de facteur XIII.

La présente invention concerne également le procédé d'obtention du concentré de fibrinogène dans lequel on soumet une fraction solubilisée de plasma contenant du fibrinogène à un traitement chimique d'inactivation virale, à une ou plusieurs étape(s) de précipitation dans une solution à un pH acide et comprenant un acide aminé (et éventuellement à un ou plusieurs traitement(s) physique(s) (traitements par rayonnement ultraviolet, en particulier UVC) ou thermique(s) d'inactivation virale).

La combinaison de ces différentes étapes permet, de manière inattendue, d'obtenir par un effet synergique le concentré de fibrinogène selon l'invention, dont la pureté est supérieure à 98 % et qui est dépourvu de contaminants viraux et dépourvu de protéinases.

Avantageusement, l'étape de précipitation à pH acide s'effectue à un pH compris entre 4.0 et 7.0, de préférence entre 5.5 et 6.5.

La concentration en acide aminé (de préférence la Glycine) dans la solution est comprise entre 0.1 et 3.3 molaire, de préférence entre 0.5 et 1.5 molaire.

En outre, le procédé de purification comporte, de préférence après chaque étape de précipitation, une ou plusieurs étape(s) de filtration du fibrinogène purifié. Préférentiellement, cette étape de filtration s'effectue sur filtre de carbone activé.

Les filtres carbone AKS-4 ou AKS-7, tels que ceux fournis par la société ZEISS, conviennent particulièrement au procédé de l'invention.

Selon l'invention, le traitement chimique d'inactivation virale consiste en un traitement par solvant-détergent tel que décrit dans la demande de brevet EP-0 131 740.

L'étape thermique d'inactivation virale s'effectue par exemple par chauffage à une température supérieure à 80 °C, pendant une durée supérieure ou égale à 10 heures, de préférence comprise entre 24 et 72 heures.

L'étape physique d'inactivation virale s'effectue par traitement de la fraction solubilisée de plasma aux rayons ultraviolets C, dont la longueur d'onde est comprise entre 250 et 270 nm, de préférence de l'ordre de 254 nm, et les doses d'irradiation sont de l'ordre de 250 Joules/m².

Selon l'invention, la fraction solubilisée de plasma est choisie parmi le groupe constitué par la fraction solubilisée d'un cryoprécipité de plasma, la fraction FI de Cohn et/ou un mélange d'entre elles.

Selon une première forme d'exécution préférée de l'invention, on soumet préalablement la fraction solubilisée d'un cryoprécipité de plasma à une étape unique de chromatographie sur une résine échangeuse d'ions.

De préférence, la résine échangeuse d'ions comporte une matrice constituée d'un gel capable, de par ses propriétés de porosité et d'hydrophobicité, de retenir le complexe facteur VIII - facteur de von Willebrand présent dans le plasma.

En outre, selon cette forme d'exécution préférée de l'invention, le procédé comporte également une étape dans laquelle on soumet préalablement la fraction solubilisée du cryoprécipité de plasma à un traitement de prépurification comprenant un traitement à l'hydroxyde d'alumine et/ou une précipitation à une température comprise entre 10 et 20 °C.

La présente invention concerne également la composition pharmaceutique et/ou cosmétique comprenant le concentré de fibrinogène selon l'invention et/ou obtenu par le procédé d'obtention selon l'invention, ledit concentré étant charactérisé en ce qu'il est dépourvu de contaminants viraux, en ce que son pureté est supérieure à 98%, et en ce qu'il est dépourvu de protéases.

En particulier, cette composition pharmaceutique et/ou cosmétique est une colle biologique telle que décrite dans la demande de brevet EP-0 305 243 et comprenant le concentré de fibrinogène selon l'invention.

### Brève description des figures.

- La figure 1: représente l'analyse densimétrique sur gel de polyacrylamide (SDS-PAGE) du fibrinogène purifié par différentes méthodes.
- Les figures 2 à 5: représentent l'analyse zymographique du fibrinogène purifié par différentes méthodes.
- La figure 6: représente de manière schématique une installation pour la préparation du concentré de fibrinogène selon l'invention.

La présente invention sera décrite de manière plus détaillée en référence aux figures annexées dans les exemples suivants donnés à titre d'illustration non limitative de l'invention.

### Exemple 1.

On utilise comme matériau de départ un cryoprécipité de plasma comprenant du fibrinogène remis en suspension.

Cette suspension de cryoprécipité est soumise à une prépurification par traitement à l'hydroxyde d'alumine et une précipitation à froid.

Cette solution prépurifiée est soumise ensuite à un traitement d'inactivation virale par solvant-détergent telle que décrite dans la demande de brevet européen EP-0 131 740.

Cette fraction solubilisée est ensuite soumise à une étape unique de chromatographie sur une résine échangeuse d'ions et on récupère sélectivement la première fraction obtenue (éluat + volume de lavage) qui est purifiée par une première étape de précipitation dans les conditions suivantes:
- température = 25 °C
- Na citrate 0,15 M, NaCl 0,15 M, Glycine 1 M
- pH 6,1 (HCl 1 normal)
- Glycine 1 M.

Ensuite, on porte la température à +4 °C et on effectue une centrifugation.

On redissout le précipité dans les conditions suivantes:
- pH 7.0
- température = 30 °C
- Na citrate 0,15 M, NaCl 0,15 M, Glycine 1 M
- filtration AKS-4 - AKS-7
- pH 6,1 (HCl 1 normal).

On reporte ensuite la température à +4 °C et on effectue une centrifugation.

Le précipité est redissout dans les conditions suivantes:
- température = 30 °C
- Na citrate 0.0 5M, monodextrose 50 g/l
- NaCl 0.05 M
- pH 7.

Le précipité est ensuite filtré sur un filtre de carbone du type AKS4-EKSP ® (SEITZ), puis concentré et diafiltré. Le produit concentré est ensuite stérifiltré après ajout stabilisateur (sucrose), mis en flacons et lyophilisé.

Les rendements obtenus sont de l'ordre de 80% et la pureté des produits atteint des valeurs de l'ordre de 98% ± 2%.

### Exemple 2.

On utilise, comme dans l'exemple 1, comme matériau de départ, un cryoprécipité de plasma soumis à un traitement chimique d'inactivation virale par solvant-détergent; mais sans soumettre le cryoprécipité à une étape de prépurification par chromatographie comme dans l'exemple 1.

La purification du fibrinogène est obtenue par une seule étape de précipitation. Les rendements obtenus sont de l'ordre de 70 à 80% et la pureté des produits atteint des valeurs de l'ordre de 75 à 85%.

Le tableau 1 ci-dessous reprend les données comparatives de différentes méthodes de purification du fibrinogène.

**Tableau 1.**

| | Rendement global % | Pureté % (e) | Tween 80 ppm (f) | TNBP ppm (g) | Protéases (h) |
|---|---|---|---|---|---|
| Methode I (a) | 70 - 80 | > 98 | < 10 | < 1 | - |
| Methode II (b)* | 70 - 80 | 75 - 85 | 125 | < 20 | ++ |
| Méthode III (c)* | > 90 | > 90 | 82 | < 10 | ± |
| Methode IV (d)* | nd | 70 - 80 | nd | nd | ++ |

| | | | | | |
|---|---|---|---|---|---|
| (a) Le fibrinogène est purifié à partir de la première fraction obtenue sélectivement à partir d'une étape unique de chromatographie, suivie de 2 précipitations à la Glycine et filtration sur filtre AKS4-EKSP ® (exemple 1). | | | | | |
| (b) Le fibrinogène est purifié à partir d'un cryoprécipité de plasma non prétraité par chromatographie et purifié par une seule précipitation (exemple 2). | | | | | |
| (c) Le procédé est identique au procédé (a) mais ne comporte qu'une seule précipitation à la Glycine. | | | | | |
| (d) Le fibrinogène est purifié selon le procédé décrit dans la demande de brevet PCT/FR89/00050. | | | | | |
| (e) Analyse densitométrique du gel de polyacrylamide (SDS-PAGE, voir figure 1). | | | | | |
| (f) La limite supérieure acceptable de la concentration du Tween 80 doit être inférieure à 100 ppm. | | | | | |
| (g) La limite supérieure acceptable de la concentration du TNBP doit être inférieure à 10 ppm. | | | | | |
| (h) La présence de protéases a été mise en évidence par analyse zymographique (voir figure 2). | | | | | |
| * exemple comparatif | | | | | |

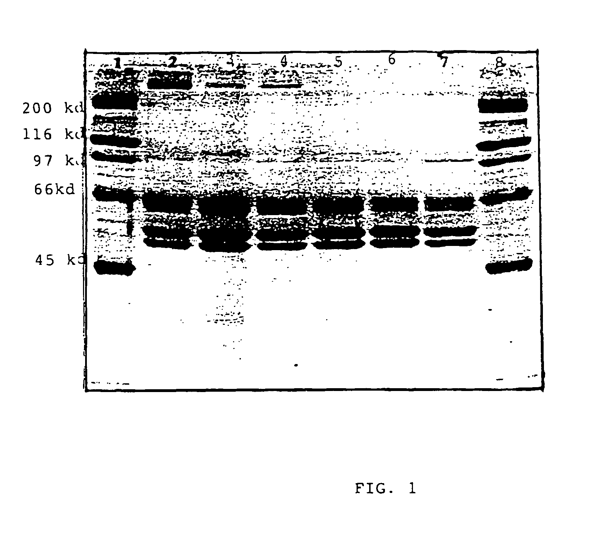

L'analyse densitométrique sur gel de polyacrylamide (SDS-PAGE) de la figure 1 reprend différents lots de fibrinogènes obtenus par différents procédés selon l'invention et selon l'état de la technique.

### Quantité de matériel déposé par piste : 5 µg protéine

- Pistes 1,8 : standards de poids moléculaires BIO-RAD ® "High range"
- Piste 2 : mise en solution du cryoprécipité
- Piste 3 : fibrinogène standard de chez Kordia ®
- Piste 4 : fibrinogène préparé à partir du cryoprécipité (2 précipitations à la Glycine)
- Piste 5 : fibrinogène préparé à partir du pic A d'une colonne échangeuse d'ions (1 précipitation à la Glycine)
- Piste 6 : fibrinogène préparé à partir du pic A d'une colonne échangeuse d'ions (2 précipitations à la Glycine)
- Piste 7 : fibrinogène préparé à partir d'une colonne Héparine-Sépharose (Pharmacia)

Les pistes 2, 3, 4 et 7 comportent une bande supplémentaire (MW 300.000) de fibronectine.

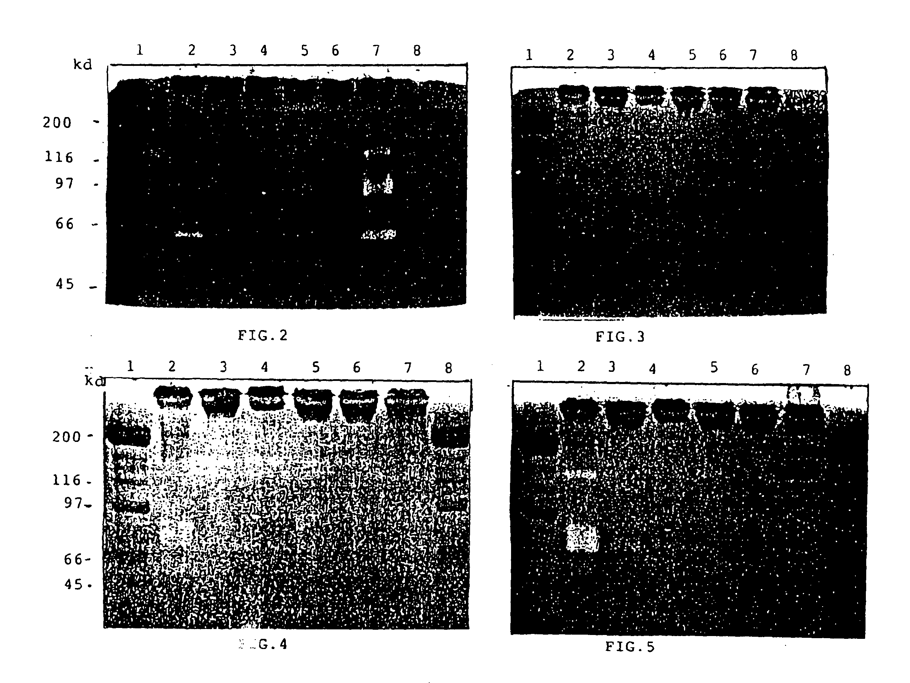

L'analyse zymographique présentée aux figures 2 à 5 reprend l'analyse de différents lots de fibrinogène obtenus par différents procédés selon l'invention et selon l'état de la technique (sur gélatine (figure 2), sur gélatine + EDTA (figure 3), sur caséine (figure 4), sur caséine + EDTA (figure 5)).

### Quantité de matériel déposé par piste : 500 µg protéine

- Pistes 1,8 : standards de poids moléculaires BIO-RAD ® "High range"
- Piste 2 : mise en solution du cryoprécipité
- Piste 3 : fibrinogène standard de chez Kordia ®
- Piste 4 : fibrinogène préparé à partir du cryoprécipité (2 précipitations à la Glycine)
- Piste 5 : fibrinogène préparé à partir du pic A d'une colonne échangeuse d'ions (1 précipitation à la Glycine)
- Piste 6 : fibrinogène préparé à partir du pic A d'une colonne échangeuse d'ions (2 précipitations à la Glycine)
- Piste 7 : fibrinogène préparé à partir d'une colonne Héparine-Sépharose (Pharmacia)

Les pistes 2 et 7 indiquent clairement la présence de protéases.

### Exemple 3 : Préparation de concentré de fibrinogène à partir de la fraction FI (fractionnement de Cohn (voir tableaux 2 et 3).

### Obtention de la fraction FI.

50 l de plasma sont décongelés à 0 ± 2 °C et centrifugés pour obtenir le cryoprécipité. Le pH du plasma pauvre en cryoprécipité est amené à pH 7.2 avec du HCl et est additionné par de l'éthanol (9 ± 1%). La température est maintenue à -2,5 °C. Après deux heures d'incubation, la suspension est centrifugée et un culot (Fraction I de Cohn ou FI) est obtenu (± 800 g).

La fraction FI est resuspendue dans du tampon citrate (citrate Na 0,15 M - NaCl 0,15 M - pH 7.0 ± 0.1) à 15 °C. De l'alhydrogel (2% concentration finale) est ajouté, et l'incubation se poursuit 20 minutes à 22 °C. Après élimination du culot riche en protéases, le surnageant est filtré sur filtre clarifiant (1 µm Pall) avec un débit de 1 l/m. Une première inactivation virale est menée par addition de solvant-détergent (8 heures en présence de Tween 80 1% et de TNBP 0,3% à 25 °C).

### Première précipitation à la Glycine.

Après inactivation, le PH est ajusté à 6.1 ± 0.1 et la concentration en Glycine est amenée à 1 M. Après minimum 2 heures de précipitation à 4 °C, la suspension est centrifugée ou décantée. Le culot est dissous dans 10 fois son volume en tampon citrate à 30°C et le pH est ramené à 7.0 ± 0.1.

### Elimination du solvant-détergent par filtration absorptive clarifiante.

La suspension est filtrée sur charbon de type AKS-4 ou AKS-7 (3 plaques) et filtre de type Kieselguhr EKSP (3 plaque) (débit 700 ml/min).

L'élimination du solvant-détergent est plus efficace sur les filtres charbon AKS-4 ou AKS-7 tels que ceux fournis par la société ZEISS que sur les filtres "delipided" tels que ceux fournis par la société CUNO.

### Seconde précipitation à la Glycine.

Le pH du filtrat est ramené à 6.1 ± 0.1, et la Glycine est additionnée (concentration finale 1 M). La précipitation à 4 °C dure minimum 2,5 heures. Le précipité est obtenu après centrifugation ou décantation.

### Seconde filtration clarifiante.

Le culot est dissous dans du tampon citrate 3 fois dilué et filtré, comme précédemment décrit.

### Concentration et diafiltration.

Le filtrat de la seconde filtration est concentré par ultrafiltration (Filtron ®, Millipore) ("clotting assay") et diafiltré contre du tampon citrate 3 fois dilué.

### Addition des stabilisateurs et seconde inactivation virale par les rayons ultraviolets.

Des stabilisateurs sont additionnés au fibrinogène purifié, le pH est ajusté à 7.0 ± 0.1 et la solution de fibrinogène peut être traitée par des rayons UVC pour pouvoir inactiver notamment les virus non enveloppés (parvovirus B19, virus de l'hépatite A et C, ...).

### Filtration stérilisante.

La solution est filtrée stérilement sur filtre Millidisk ® 0,45 µm et 0,22 µm (Millipore).

### Lyophilisation et traitement thermique sévère.

Après lyophilisation, le fibrinogène peut être chauffé à 80 °C pendant une durée supérieure ou égale à 10 heures, de préférence pendant une durée comprise entre 24 et 72 heures.

### Résultats.

Le produit obtenu est caractérisé par les propriétés suivantes :
- rendement : 0,4 à 1 g/l de plasma de départ (fraction FI)
- effluent : 0,2 mg/l de plasma
- pureté : supérieure à 98%
- haute concentration en facteur VIII (même après chauffage à sec sévère)
- absence de protéases (notamment de protéases dépendantes de la vitamine K) mesurables
- produit soluble en moins de 10 minutes
- produit caractérisé par une très grande stabilité en solution : pas de coagulation après conservation pendant plus de 12 mois à 4 °C
- très bas coût de production
- faible réduction de l'activité du fibrinogène ayant subi trois inactivations virales

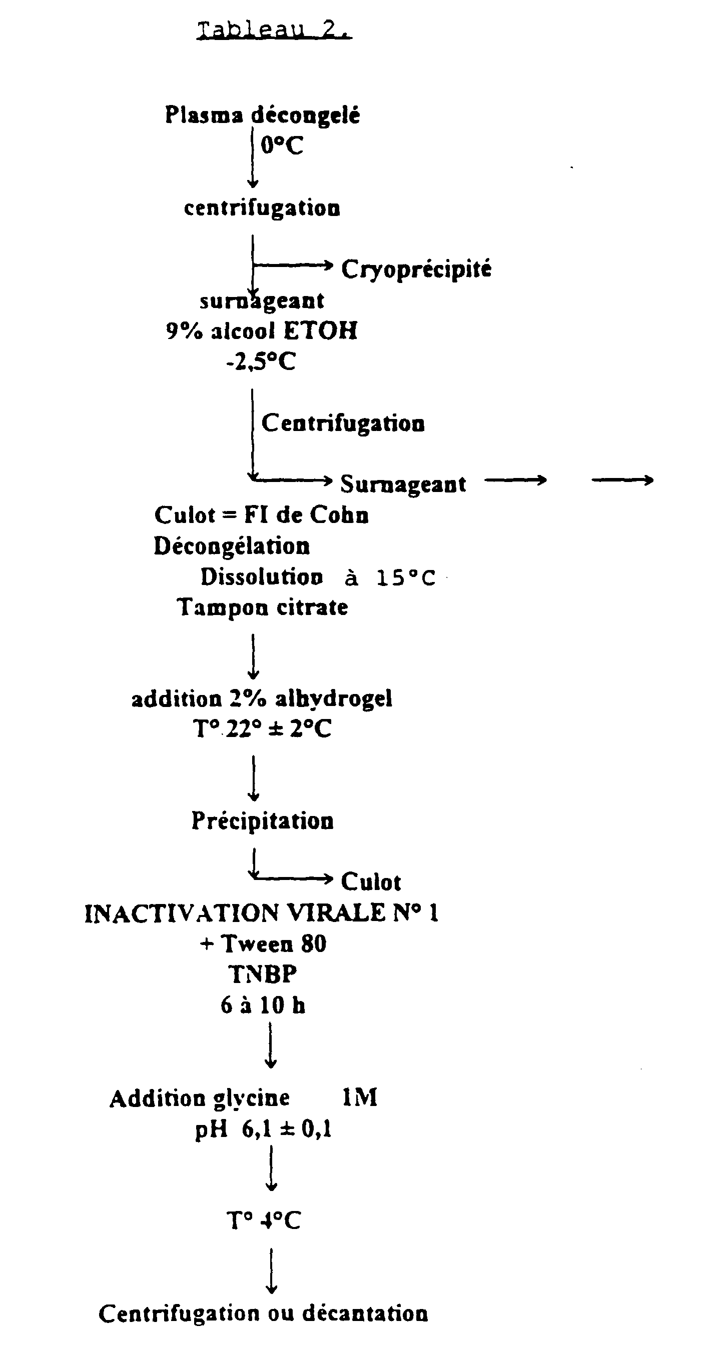

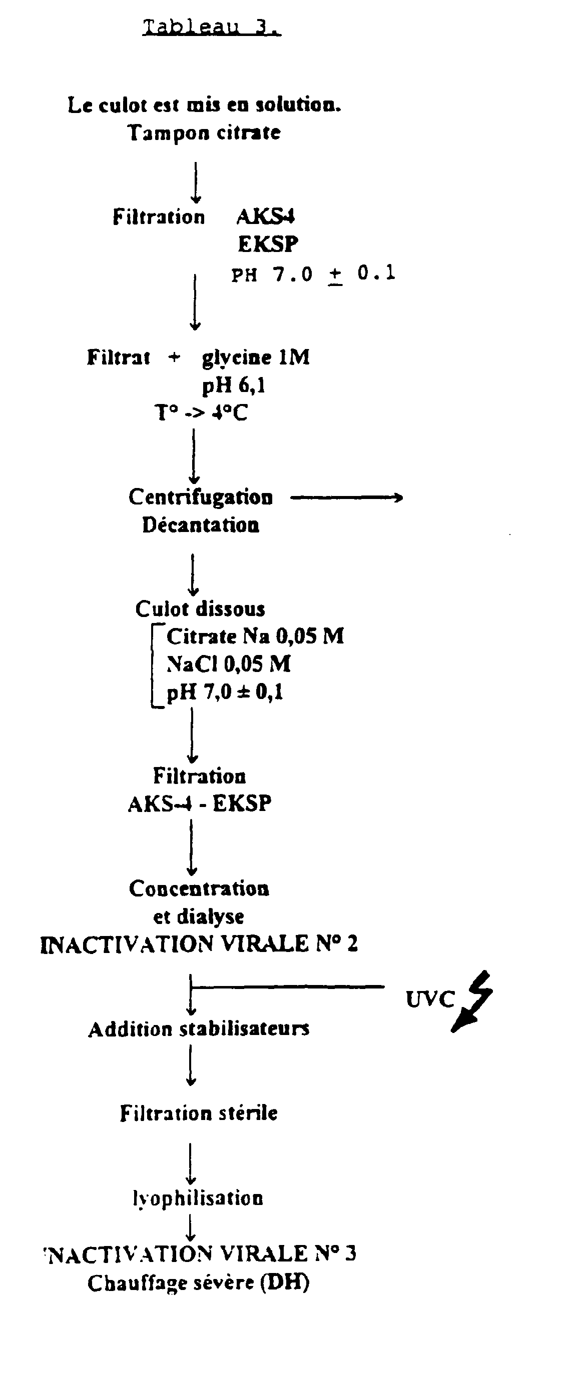

### Installation pour la préparation du concentré de fibrinogène selon l'invention.

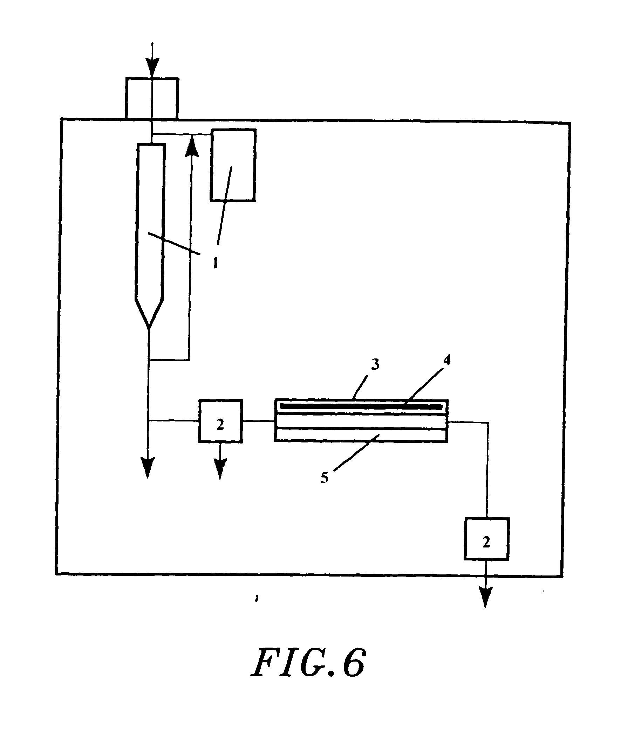

La figure 6 annexée représente de manière schématique une installation pour la préparation du concentré de fibrinogène selon l'invention.

Cette installation comprend des dispositifs (1 et 2) assurant la précipitation, la centrifugation/décantation, filtration, concentration et dialyse du concentré de fibrinogène selon l'invention et adaptable par l'homme du métier en fonction du dérivé sanguin traité.

En outre, cette installation comporte un dispositif (portant le repère de référence 3 dans la figure 6 annexée) assurant par un traitement physique une inactivation virale du dérivé sanguin.

L'installation selon la présente invention peut donc être utilisée pour un traitement physique d'inactivation virale de tout dérivé sanguin, en particulier celle des facteurs de coagulation (facteur VIII, facteur IX, ...), des immunoglobulines, de l'albumine, du fibrinogène ... .

Ce dispositif comprend un tube de désinfection UV dont plus de 90% de l'émission s'effectue entre 250 et 270 nm, de préférence à 254 nm, et qui est monté dans une enceinte réflectante qui renvoie le rayonnement vers un tube en quartz ou en matériau polymérisé et non absorbant dans cette zone de longueur d'onde. Aucun contact n'est possible entre le produit circulant dans le tube 4 et la lampe UV 5.

Un système de turbulence, tel qu'une baffle ou une injection d'azote, permet de maintenir un flux homogène dans le tube. Le système de contrôle de la quantité d'UV qui irradie le tube est placé du côté opposé du tube par rapport à la lampe. Le temps de maintien du produit peut être ajusté pour obtenir une dose constante d'irradiation. Le diamètre du tube peut être adapté au volume à traiter ainsi que la puissance ou la longueur de la lampe de désinfection. La température est contrôlée et enregistrée tant à l'intérieur de l'enceinte que dans le liquide.

Tout le système est en matériaux en accord avec les bonnes pratiques de production pharmaceutique (GMP), comme de l'inox 304, du Téflon, ..., et peut être traité de manière sanitaire sur place.

L'utilisation d'un tel système de rayonnement UV, en particulier de rayonnement UVC, permet d'inactiver les virus, notamment les virus non enveloppés, en particulier les virus simple brin tels que le parvovirus murin (5 à 6 log d'inactivation virale).

Le système est placé en aval du procédé de préparation du dérivé sanguin, par exemple avant la filtration stérilisante ou après l'ultrafiltration.

La puissance de la lampe UV est comprise entre 18 et 132 Watts. L'activité des préparations (facteur VIII, fibrinogène et IgG) utilisées est peu affectée (5% en moyenne de réduction d'activité).

Le dispositif peut être construit d'un seul tenant ou en modules juxtaposé placés en série. Les doses d'irradiation varient entre 100 et 2000 Joules/m², mais sont de préférence de l'ordre de 250 Joules/m².

Le dispositif de désinfection UV utilisé dans l'installation selon l'invention est de type s.p.1 (AQUAFINE ®, Valencia, CA (USA)).

### Exemple 5 : Analyse du concentré de fibrinogène selon l'invention.

Le tableau 4 donne une analyse des caractéristiques du concentré de fibrinogène préparé à partir d'une fraction solubilisée de plasma constituée par la fraction solubilisée d'un cryoprécipité de plasma soumis à une étape unique de chromatographie sur une résine échangeuse d'ions (effluent chromato), éventuellement traitée par un chauffage sévère (effluent chromato DH) ou obtenue par le traitement de la fraction I de Cohn (FI), éventuellement traitée par un chauffage sévère (FI DH).

Cette analyse montre que le produit obtenu présente une pureté particulièrement élevée, supérieure à 98%, présente de très faibles proportions de solvants / détergents, mais conserve une fraction importante de facteur XIII assurant l'application du concentré de fibrinogène selon l'invention en tant que colle biologique.

**Tableau 4.**

| **Fraction de départ** | **Pureté (%)** | **mg FXIII/l** | **ppm Tween 80** | **ppm TNBP** |
|---|---|---|---|---|
| Effluent chromato | >98 | 35 | 13 | <0,5 |
| Effluent chromato DH | >98 | 0,7 | 17 | <0,5 |
| FI | >98 | 360 | <10 | <0,5 |
| FI DH | >98 | 103 | <10 | <0,5 |
| DH : dry heat = chauffage sévère | | | | |

## Revendications

1. Procédé d'obtention du concentré de fibrinogène **caractérisé en ce qu'**il est dépourvu de contaminants viraux, **en ce que** sa pureté est supérieure à 98%, et **en ce qu'**il est dépourvu de protéases, **caractérisé en ce que** l'on soumet une fraction solubilisée de plasma comprenant du fibrinogène à un traitement chimique d'inactivation virale par addition de solvant/détergent, à deux ou plusieurs étapes de précipitation dans une solution comprenant un acide aminé, de préférence la Glycine, et à pH acide, et une ou plusieurs étape(s) de filtration du fibrinogène purifié, de préférence sur filtre de carbone activé.

2. Procédé selon la revendication 1, **caractérisé en ce que** le pH de la solution est compris entre 4.0 et 7.0, de préférence entre 5.5 et 6.5, et **en ce que** la concentration en acide aminé dans la solution est comprise entre 0.1 et 3.3 molaire, de préférence entre 0.5 et 1.5 molaire.

3. Procédé selon la revendication 1 ou 2, **caractérisé en ce qu'**il comporte une étape thermique d'inactivation virale, de préférence par chauffage de la fraction solubilisée de plasma à une température supérieure ou égale à 80°C pendant une durée supérieure ou égale à 10 heures, et/ou une étape physique d'inactivation virale par traitement de la fraction solubilisée de plasma aux rayons ultraviolets C, dont la majorité de l'émission du rayonnement s'effectue de préférence entre 250 et 270 nm, et dans laquelle les doses d'irradiation sont de préférence de l'ordre de 250 Joules/m².

4. Procédé selon l'une quelconque des revendications 1 à 3, **caractérisé en ce que** la fraction solubilisée du plasma est choisie parmi le groupe constitué par la fraction solubilisée d'un cryoprécipité de plasma, la fraction FI de Cohn et/ou un mélange d'entre elles.

5. Procédé selon la revendication 4, **caractérisé en ce que** la fraction solubilisée du cryoprécipité de plasma est préalablement soumise à une étape unique de chromatographie sur une résine échangeuse d'ions, comportant de préférence une matrice constituée d'un gel capable de, par ses propriétés de porosité et d'hydrophobicité de retenir le complexe facteur VIII - facteur de von Willebrand.

6. Procédé selon la revendication 5, **caractérisé en ce que** l'on soumet préalablement la fraction solubilisée du cryoprécipité de plasma à un traitement de prépurification comprenant un traitement à l'hydroxyde d'alumine et/ou une précipitation à une température comprise entre 10 et 20°C.

7. Composition pharmaceutique, cosmétique ou colle biologique comprenant un concentré de fibrinogène, **caractérisé en ce qu'**il est dépourvu de contaminants viraux, **en ce que** sa pureté est supérieure à 98%, et **en ce qu'**il est dépourvu de protéases.

## Claims

1. A process for obtaining the fibrinogen concentrate, **characterized in that** it is free of viral contaminants, the purity thereof is higher than 98%, and it is free of proteases, **characterized in that** a fibrinogen-comprising plasma solubilised fraction is submitted to a chemical treatment of viral inactivation by addition of solvent/detergent, to one or more precipitation steps in a solution comprising an amino acid, preferably glycine, and at an acidic pH, and to one or more filtration steps for the purified fibrinogen, preferably on an active carbon filter.

2. A process according to claim 1, **characterized in that** the solution pH ranges between 4.0 and 7.0, preferably between 5.5 and 6.5, and the amino acid concentration in the solution ranges between 0.1 and 3. 3 molar, preferably between 0.5 and 1.5 molar.

3. A process according to claim 1 or 2, **characterized in that** it includes a thermal step of viral inactivation, preferably by heating the solubilized plasma fraction at a temperature higher than or equal to 80°C, for 10 or more hours, and/or a physical step of viral inactivation by treating the solubilized plasma fraction to ultraviolet C rays, most of the radiation emission of which is carried out preferably between 250 and 270 nm, and wherein the radiation doses are preferably of about 250 Joules/m².

4. A process according to any of claims 1 to 3, **characterized in that** the solubilized plasma fraction is selected among the group comprising the solubilized fraction of a plasma cryoprecipitate, Cohn's FI fraction and/or a mixture thereof.

5. A process according to claim 4, **characterized in that** the solubilized fraction of the plasma cryoprecipitate is previously submitted to a single chromatography step on an ion exchanger resin, comprising preferably a matrix made of a gel capable through the porosity and hydrophobicity properties thereof to keep the factor VIII-von Willebrand factor complex.

6. A process according to claim 5, **characterized in that** the solubilized fraction of the plasma cryoprecipitate is previously submitted to a prepurification treatment including an alumina hydroxide treatment and/or a precipitation at a temperature ranging between 10 and 20°C.

7. A pharmaceutical composition, cosmetic or biological glue comprising the fibrinogen concentrate, **characterized in that** it is free of viral contaminants, the purity thereof is higher than 98%, and it is free of proteases.

## Patentansprüche

1. Verfahren zur Herstellung des Fibrinogenkonzentrats, **dadurch gekennzeichnet, daß** es frei von viralen Verunreinigungen ist, daß seine Reinheit größer als 98% ist, und daß es frei von Proteasen ist, **dadurch gekennzeichnet, daß** eine solubilisierte Plasmafraktion, die Fibrinogen enthält, einer chemischen Behandlung zur viralen Inaktivierung unterworfen wird durch Zugabe von Lösungsmittel/Reinigungsmittel bei einem oder mehreren Schritten zur Ausfällung in einer Lösung, die eine Aminosäure, vorzugsweise Glycin, enthält, und bei saurem pH, und einem oder mehreren Schritten zur Filtration des gereinigten Fibrinogens, vorzugsweise auf Aktivkohlefilter.

2. Verfahren gemäß Anspruch 1, **dadurch gekennzeichnet, daß** der pH der Lösung zwischen 4,0 und 7,0, vorzugsweise zwischen 5,5 und 6,5 liegt, und daß die molare Konzentration an Aminosäure in der Lösung zwischen 0,1 und 3,3, vorzugsweise zwischen 0,5 und 1,5 liegt.

3. Verfahren gemäß Anspruch 1 oder 2, **dadurch gekennzeichnet, daß** es einen thermischen Schritt zur viralen Inaktivierung umfaßt, vorzugsweise durch Erhitzen der solubilisierten Plasmafraktion auf eine Temperatur von 80°C oder darüber während einer Dauer von mindestens 10 Stunden, und/oder einen physikalischen Schritt zur viralen Inaktivierung umfaßt durch Behandlung der solubilisierten Plasmafraktion mit Ultraviolett-C-Strahlen, wo die meiste Strahlung vorzugsweise zwischen 250 und 270 nm emittiert wird, und bei dem die Bestrahlungsdosen vorzugsweise ungefähr 250 Joule/m² betragen.

4. Verfahren gemäß irgendeinem der Ansprüche 1 bis 3, **dadurch gekennzeichnet, daß** die solubilisierte Plasmafraktion in der Gruppe gewählt wird, die von der solubilisierten Fraktion eines Plasma-Kryopräzipitats, der FI-Fraktion von Cohn und/oder einem Gemisch davon gebildet wird.

5. Verfahren gemäß Anspruch 4, **dadurch gekennzeichnet, daß** die solubilisierte Fraktion des Plasma-Kryopräzipitats zuvor einem einzelnen Chromatographieschritt auf einem Ionenaustauscherharz unterworfen wird, das vorzugsweise eine Matrix umfaßt, die aus einem Gel besteht, das aufgrund seiner porösen und hydrophoben Eigenschaften den Komplex Faktor VIII-von Willebrand-Faktor zurückhalten kann.

6. Verfahren gemäß Anspruch 5, **dadurch gekennzeichnet, daß** die solubilisierte Fraktion des Plasma-Kryopräzipitats zuvor einer Vorreinigungsbehandlung unterworfen wird, die eine Behandlung mit Aluminiumhydroxid und/oder eine Ausfällung bei einer Temperatur zwischen 10 und 20°C aufweist.

7. Pharmazeutische, kosmetische Zusammensetzung oder biologischer Kleber, die das Fibrinogenkonzentrat aufweisen, **dadurch gekennzeichnet, daß** es frei von viralen Verunreinigungen ist, daß seine Reinheit größer als 98% ist, und daß es frei von Proteasen ist.
